# EUROPEAN PATENT APPLICATION

(11) **EP 4 728 990 A1**
(43) Date of publication of application: **22.04.2026**
(21) Application number: 25204012.6
(22) Date of filing: 23.09.2025
(51) Int. Cl.: A61B 6/58

(54) **IMAGING SYSTEM PHANTOM**

(30) Priority: 18.10.2024 US 202418920793
(71) Applicant: GE Precision Healthcare LLC, Waukesha, WI 53188 (US)
(72) Inventor: KIM, Changlyong, Waukesha, 53188-1696 (US); CEDERSTRÖM, Björn, 10691 Stockholm (SE); COLLIN, Daniel, 114 21 Stockholm (SE); TRAN, Vi-Hoa, Waukesha, 53188-1696 (US); BOUDRY, John M., Waukesha, 53188-1696 (US); DELONG SAMALIK, Michelle Marie Severino, Waukesha, 53188-1696 (US); EVANGELIST, Matthew J., Waukesha, 53188-1696 (US); SMITH, Brandon A., Waukesha, 53188-1696 (US); LEWIS, Chelsey Amanda, Waukesha, 53188-1696 (US); BERRIOS COLLAZO, Angel, Waukesha, 53188-1696 (US)
(74) Representative: AWA Sweden AB

(57) **Abstract**

A retention system for a phantom for calibrating an imaging system, the retention system comprising at least one rod holder, wherein the at least one rod holder includes at least one opening, wherein the at least one opening is configured to secure at least one rod of the phantom in the retention system, and a fastener, wherein the fastener is configured to secure the at least one rod holder to a body of the phantom.

## Description

### TECHNICAL FIELD

Embodiments of the subject matter disclosed herein relate to a phantom, and more particularly, to a phantom used to calibrate a photon counting computed tomography (CT) scanner.

### BACKGROUND

In computed tomography (CT) imaging systems, an electron beam generated by a cathode is directed towards a target within an X-ray tube. A fan-shaped or cone-shaped beam of X-rays produced by electrons colliding with the target is directed towards a subject, such as a patient. After being attenuated by the object, the X-rays impinge upon an array of X-ray detectors, generating an image. One example of a CT system is a Photon Counting CT (PCCT), where the X-ray detectors are photon-counting detectors, and photons are counted to provide spectral information. A calibration process may be performed periodically on the PCCT system to obtain projection data of materials that simulate varying human tissue densities. The calibration process may include performing a CT imaging procedure on an object, referred to as a phantom.

### SUMMARY

In one example, a retention system for a phantom for calibrating an imaging system includes at least one rod holder, wherein the at least one rod holder includes at least one opening, wherein the at least one opening is configured to secure at least one rod of the phantom in the retention system, and a fastener, wherein the fastener is configured to secure the at least one rod holder to a body of the phantom.

The above advantages and other advantages and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings.
FIG. 1 shows a pictorial view of a computed tomography (CT) imaging system, in accordance with one or more embodiments of the present disclosure.
FIG. 2 shows a block schematic diagram of an example CT imaging system, in accordance with one or more embodiments of the present disclosure.
FIG. 3 shows a side view of an example phantom including a rod retention system according to an embodiment of the present disclosure.
FIG. 4 shows a front view of the example phantom of FIG. 3 according to an embodiment of the present disclosure.
FIG. 5 shows a front view of a holder of the rod retention system of the phantom of FIG. 3 according to an embodiment of the present disclosure.
FIG. 6 shows a perspective view of a portion of the phantom of FIG. 3 configured to receive the holder according to an embodiment of the present disclosure.
FIG. 7 shows a holder for a rod coupled to a phantom according to a second embodiment of the present disclosure.
FIG. 8 is a perspective view of a phantom comprising end plates configured to retain rods according to an embodiment of the present disclosure.
FIG. 9 shows a perspective view of a phantom including another rod retention system according to an embodiment of the present disclosure.
FIG. 10 is a first view of an example opening used in conjunction with a loop for attaching the example rod retention system of FIG. 9 to a phantom body according to one or more embodiments of the present disclosure.
FIG. 11 is a second view of the opening of FIG. 10 for attaching the rod retention system of FIG. 9 to a phantom body according to one or more embodiments of the present disclosure.
FIG. 12 shows a first side-on view of the phantom of FIG. 9.
FIG. 13 shows a second side-on view of the phantom of FIG. 9.
FIG. 14 shows a front face-on view of the phantom of FIG. 9.
FIG. 15 shows a back side view of the phantom of FIG. 9.
FIG. 16 shows a perspective view of a rod retention system.
FIG. 17 shows a perspective view of an example rod retention system.
FIG. 18 shows a perspective view of a rod retention system for a phantom according to an embodiment of the present disclosure.
FIG. 19 shows a perspective view of a rod retention system for a phantom according to an embodiment of the present disclosure.
FIG. 20 shows a perspective view of a rod retention system for a phantom according to an embodiment of the present disclosure.
FIG. 21 shows a perspective view of a rod retention system for a phantom according to an embodiment of the present disclosure.
FIG. 22 shows a perspective view of a rod retention system for a phantom according to an embodiment of the present disclosure.
FIG. 23 shows a perspective view of a rod retention system for a phantom according to an embodiment of the present disclosure.
FIG. 24 shows a perspective view of a rod retention system for a phantom according to an embodiment of the present disclosure.
FIG. 25 shows a perspective view of an embodiment of an iodine rod configured for one or more of the phantoms of the present disclosure.
FIG. 26 shows a perspective view of another embodiment of an iodine rod including a rod holder configured for one or more of the phantoms of the present disclosure.

### DETAILED DESCRIPTION

The description and embodiments of the subject matter disclosed herein relate to a phantom for calibration scans of an imaging system, such as a photon counting computed tomography (PCCT) system. Imaging systems, such as PCCT systems, may demand regular calibration scans, such as daily or weekly calibration scans to offset any gain drift, realized from hardware such as X-ray focal spot position change, or radiation degradation of the detectors. Further, PCCT systems may obtain spectral information that allows generation of basis material decomposition (BMD) images. Calibrating PCCT systems may demand scans of different solutions. Scanning these different solutions may be difficult and increase systems costs.

Thus, embodiments are disclosed herein for a phantom for calibrating imaging systems such as PCCT systems, dual-energy CT (DECT) systems, or other CT systems. The phantom disclosed herein may include a main portion of the phantom (e.g., a phantom body) and one or more rods containing an iodinated solution within each rod. The rod(s) may be retained or attached to the phantom body via a retention system. In one example, the phantom body is a water phantom. The retention system may include retaining features shaped onto auxiliary features that are configured to interface with features of the water phantom. Additionally, or alternatively, the retention system may include a fastening device configured to couple to an exterior of the phantom body. The material within the rod may include a single element or a hybrid fluid including a plurality of different elements such as iodine or calcium. The material within the rod coupled to the outside of the phantom body may be different than a material retained within the phantom body.

The phantom or a surface of the phantom body of the present disclosure may include a groove or other feature that receives a protrusion of the retention system. Additionally, or alternatively, the phantom may include end plates with one or more eyelets extending radially outward therefrom. The eyelets of the endplates may align along a longitudinal axis of the phantom so that the rods may be held by the eyelets. The example endplates may be attached to the phantom body or integrally formed with the phantom body.

In another example, the retention system may be configured as a retrofit assembly such that the retention system may be adapted to couple to pre-existing phantom configurations. The retention system may be configured to couple to an exterior of the phantom or phantom body. The retention system may include flexible materials that simplify coupling the retention system and iodinated rods to the phantom body. The embodiments of the different retention systems are described in greater detail below.

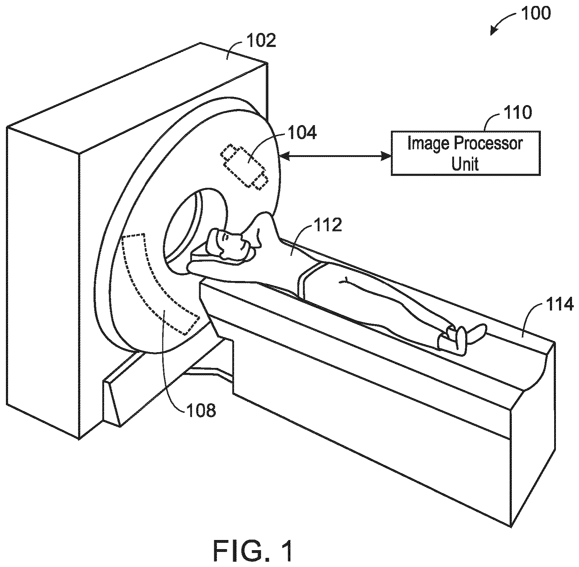

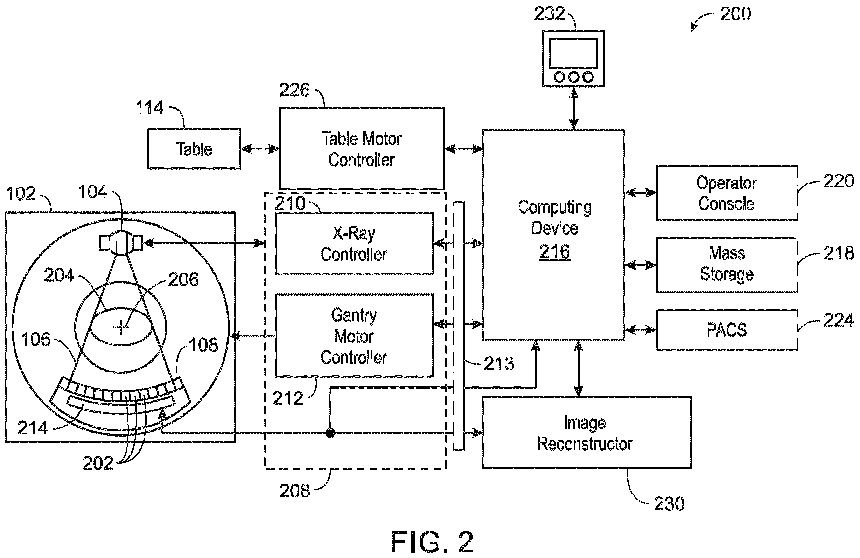

FIG. 1 illustrates an exemplary PCCT system 100 (also referred to as a photon counting X-ray imaging system) configured for CT imaging with photon counting detectors. Particularly, the PCCT system 100 is configured to image a subject 112 such as a patient, an inanimate object, one or more manufactured parts, and/or foreign objects such as dental implants, stents, and/or contrast agents present within the body. The PCCT system 100 includes a gantry 102, which in turn, may further include at least one X-ray source 104 configured to project a beam of X-ray radiation 106 (see FIG. 2) for use in imaging the subject 112 laying on a table 114. Specifically, the X-ray source 104 is configured to project the X-ray radiation beams 106 towards a detector array 108 positioned on the opposite side of the gantry 102. Although FIG. 1 depicts a single X-ray source 104, in certain embodiments, multiple X-ray sources and detectors may be employed to project a plurality of X-ray radiation beams for acquiring projection data at the same or different energy levels corresponding to the patient. In some embodiments, the X-ray source 104 may enable dual-energy spectral imaging by rapid peak kilovoltage (kVp) switching. In the embodiments described herein, the X-ray detector employed is a photon counting detector which is capable of differentiating X-ray photons of different energies.

In certain embodiments, the PCCT system 100 further includes an image processor unit 110 configured to reconstruct images of a target volume of the subject 112 using an iterative or analytic image reconstruction method. For example, the image processor unit 110 may use an analytic image reconstruction approach such as filtered back projection (FBP) to reconstruct images of a target volume of the patient. As another example, the image processor unit 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 112. In some examples the image processor unit 110 may use an analytic image reconstruction approach such as FBP in addition to an iterative image reconstruction approach.

In some CT imaging system configurations, an X-ray source projects a cone-shaped X-ray radiation beam which is defined with respect to an X-Y-Z Cartesian coordinate system and generally referred to as an "imaging volume." The X-ray radiation beam passes through an object being imaged, such as the patient or subject. The X-ray radiation beam, after being attenuated by the object, impinges upon an array of detector elements. The intensity of the attenuated X-ray radiation beam received at the detector array is dependent upon the attenuation of an X-ray radiation beam by the object. Each detector element of the array produces a separate electrical signal that is a measurement of the X-ray beam attenuation at the detector location. The attenuation measurements from all the detector elements are acquired separately to produce a transmission profile.

In some CT systems, the X-ray source and the detector array are rotated with a gantry within the imaging volume and around the object to be imaged such that an angle at which the X-ray beam intersects the object constantly changes. A group of X-ray radiation attenuation measurements, e.g., projection data, from the detector array at one gantry angle is referred to as a "view." A "scan" of the object includes a set of views made at different gantry angles, or view angles, during one revolution of the X-ray source and detector.

FIG. 2 illustrates an exemplary imaging system 200 similar to the PCCT system 100 of FIG. 1. In accordance with aspects of the present disclosure, the imaging system 200 is configured for imaging a subject 204 (e.g., the subject 112 of FIG. 1). During certain scans, the subject may be a phantom. A phantom may be an object configured to be scanned by the PCCT system as part of a calibration process for the PCCT system. In one embodiment, the imaging system 200 includes the detector array 108 (see FIG. 1). The detector array 108 further includes a plurality of detector elements 202 that together sense the X-ray radiation beam 106 (see FIG. 2) that passes through the subject 204 (such as a patient) to acquire corresponding projection data. In some embodiments, the detector array 108 may be fabricated in a multi-slice configuration including the plurality of rows of cells or detector elements 202, where one or more additional rows of the detector elements 202 are arranged in a parallel configuration for acquiring the projection data. The detector elements 202 may also be referred to as pixels or detector pixels.

In certain embodiments, the imaging system 200 is configured to traverse different angular positions around the subject 204 for acquiring desired projection data. Accordingly, the gantry 102 and the components mounted thereon may be configured to rotate about a center of rotation 206 for acquiring the projection data, for example, at different energy levels. Alternatively, in embodiments where the projection angle relative to the subject 204 varies as a function of time, the mounted components may be configured to move along a general curve rather than along a segment of a circle.

As the X-ray source 104 and the detector array 108 rotate, the detector array 108 collects data of the attenuated X-ray beams. The data collected by the detector array 108 undergoes pre-processing and calibration to condition the data to represent the line integrals of the attenuation coefficients of the scanned subject 204. The processed data are commonly called projections. In some examples, the individual detectors or detector elements 202 of the detector array 108 may include photon counting detectors which register the interactions of individual photons into one or more energy bins.

The acquired sets of projection data may be used for basis material decomposition (BMD). During BMD, the measured projections are converted to a set of material-density projections. The material-density projections may be reconstructed to form a set of material-density maps or images of each respective basis material, such as bone, soft tissue, and/or contrast agent maps. The density maps or images may be, in turn, associated to form a 3D volumetric image of the basis material, for example, bone, soft tissue, and/or contrast agent, in the imaged volume.

Once reconstructed, the basis material image produced by the imaging system 200 reveals internal features of the subject 204, expressed in the densities of two basis materials. The density image may be displayed to show these features. In traditional approaches to diagnosis of medical conditions, such as disease states, and more generally of medical events, a radiologist or physician would consider a hard copy or display of the density image to discern characteristic features of interest. Such features might include lesions, sizes and shapes of particular anatomies or organs, and other features that would be discernable in the image based upon the skill and knowledge of the individual practitioner.

In one embodiment, the imaging system 200 includes a control mechanism 208 to control movement of the components such as rotation of the gantry 102 and the operation of the X-ray source 104. In certain embodiments, the control mechanism 208 further includes an X-ray controller 210 configured to provide power and timing signals to the X-ray source 104. Additionally, the control mechanism 208 includes a gantry motor controller 212 configured to control a rotational speed and/or position of the gantry 102 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate data from a subset of the detector elements 202 into so-called macro-detectors. The data sampled and digitized by the DAS 214 is transmitted to a computer or computing device 216 via a slip ring 213. In one example, the computing device 216 stores the data in a storage device or mass storage 218. The storage device 218, for example, may be any type of non-transitory memory and may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the X-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown) or a touchscreen to allow the operator to specify the commands and/or scanning parameters.

Although FIG. 2 illustrates one operator console 220, more than one operator console may be coupled to the imaging system 200, for example, for inputting or outputting system parameters, requesting examinations, plotting data, and/or viewing images. Further, in certain embodiments, the imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a table 114 which may be a motorized table. Specifically, the table motor controller 226 may move the table 114 for appropriately positioning the subject 204 in the gantry 102 for acquiring projection data corresponding to the target volume of the subject 204.

As previously noted, the DAS 214 samples and digitizes the projection data acquired by the detector elements 202. Subsequently, an image reconstructor 230 uses the sampled and digitized X-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the imaging system 200 and instead the computing device 216 may perform one or more functions of the image reconstructor 230. Moreover, the image reconstructor 230 may be located locally or remotely, and may be operatively connected to the imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 to generate useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

Information may be transmitted between the components residing in the gantry 102 and external devices (such as the computing device 216 and/or image reconstructor 230) via the slip ring 213, which facilitates electronic communication across the rotating gantry. In some examples, the gantry and internal components (e.g., the control mechanism 208, X-ray source 104, the detector array 108) may be collectively defined as a PCCT scanner, and as such the computing device 216 and image reconstructor 230 may reside off the scanner.

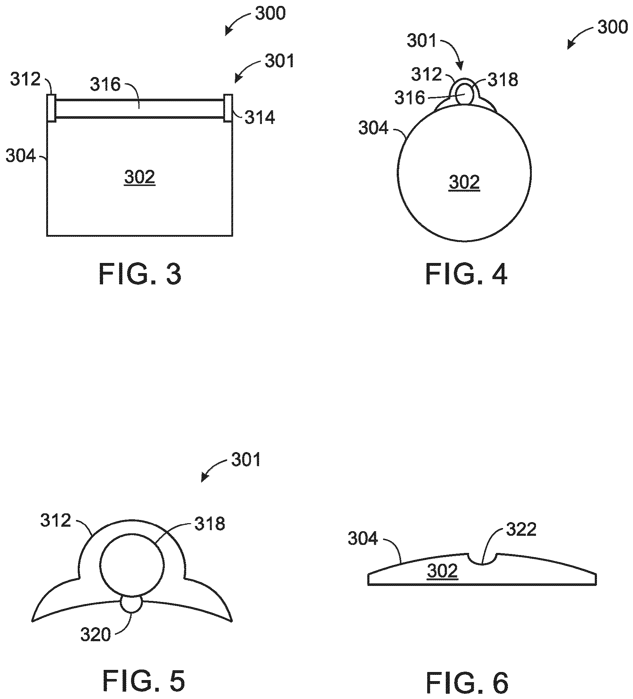

Turning now to FIGS. 3, 4, 5, and 6, they show a first embodiment of a phantom 300 including an example rod retention system 301. The phantom 300 may also include a body 304 made of a first material 302, which may be contained within the body 304. The side view of FIG. 3 shows a first rod holder 312 and a second rod holder 314 of the rod retention system 301. A rod 316 may extend from the first rod holder 312 to the second rod holder 314. The rod 316 may extend along an outer surface of the body 304 parallel with a central or longitudinal axis of the body.

The first rod holder 312 and the second rod holder 314 may be identical to one another in shape and size. The first rod holder 312 may be coupled to an external surface of the body 304 at a first end of the phantom 300. The second rod holder 314 may be coupled to an external surface of the body 304 at a second end, opposite the first end, of the phantom 300. The rod 316 may extend a length of the body 304 from the first rod holder 312 to the second rod holder 314. In one example, the length the rod 316 extends is the entire length of the body 304.

In the illustrated examples herein, the body 304 of the phantom may be visible in areas where the first rod holder 312 and the second rod holder 314 are not coupled to the body. In this way, the first rod holder 312 and the second rod holder 314 may cover the body 304 only at the areas proximal to ends. In other examples, the phantom, including the body, may be covered with a protective material to prevent damage to the phantom, for example, while a technician is handling the phantom.

A face-on view of the phantom 300 is shown in FIG. 4. As illustrated, the first rod holder 312 may include at least one surface that corresponds to a shape of the body 304. That is, in examples where the body 304 is a cylindrical shape, a surface of the rod holder 312 in contact with the body 304 may include a curved shape that matches a curvature of the body 304. As discussed above, the second rod holder 314 is substantially similar to the first rod holder 312. As such, the first rod holder 312 and the second rod holder 314 are flush with the exterior surface of the body 304. In some examples, changes may be made to the shape of the first or second rod holder 312, 314 based corresponding with differences in the location of the body to which each corresponding rod holder 312, 314 is coupled such that each rod holder 312, 314 includes at least on surface in contact with and corresponding to the shape of the body.

The first rod holder 312 may include an opening 318. The opening 318 may include a circular cross-sectional shape. In other examples, the opening may have another shape, including but not limited to oblong, semi-circular, square, rectangular, or may have a combination of curved and flat surfaces corresponding to surfaces of a rod. The rod 316 may be inserted through the opening 318. In one example, the opening 318 may be adjacent to the body 304. In this way, the rod 316 may be proximal to the body 304. In one example, the rod 316 may touch a surface of the body 304 when positioned in the opening 318. In one example, the opening 318 may shape an eyelet of the first rod holder 312, wherein the eyelet is configured to receive and retain the rod 316. In one example, the body 304 may include a first material 302 and the rod 316 may include a second material, wherein the second material is different than the first material. In one example, the first material is non-iodinated and the second material is iodinated. As such, the first material may not include iodine and the second material may include iodine.

FIG. 5 shows a face-on view of the first rod holder 312. The first rod holder 312 may include a protrusion 320 that extends from the opening 318. The protrusion functions as a fastener to attach the example first rod holder 312 to the body 304 of the phantom 300 (i.e., in conjunction with the notch 322 described in more detail below). The example protrusion 320 may include a circular shape. In some examples, additionally or alternatively, the protrusion 320 may include other shapes such as a square shape, a star shape, a trapezoid shape, or other shape without departing from the scope of the present disclosure.

FIG. 6 shows a detailed partial view of the body 304 at a location where the first rod holder 312 may be coupled to the body 304. The body 304 may include a notch 322. The notch 322 may include a circular shape that is complementary to the protrusion 320 of the first rod holder 312. Alternatively, the notch 322 is shaped to correspond to any other shape of the protrusion 320. The protrusion 320 may slide into the notch 322 such that the first rod holder 312 is coupled to the body 304.

A length of the notch 322 may be based on a length of the protrusion 320 of the first rod holder 312. In one example, the length of the notch 322 is equal to the length of the protrusion 320 such that the first rod holder 312 is flush with a leading edge of the first end of the phantom 300. Alternatively, a length of the notch 322 corresponds to a length of the body 304 of the phantom 300 such that a first rod holder 312 and a second rod holder 314 can be coupled with the notch at opposite ends of the body to enable alignment of the first rod holder 312 and the second rod holder 314. Additionally, the notch 322 may provide a fixed location for the first rod holder 312 in order to orient the first rod holder 312 in a desired position. The protrusion 320 may be inserted into the notch 322. The protrusion 320 may interlock with the notch 322 and retain the first rod holder 312. In some examples, the phantom body 304 may include a plurality of notches 322, each of which may be coupled with a protrusion of a rod holder, such as the first rod holder 312 and/or a second rod holder 314, such that multiple rods 316 can be attached to the body 304 of the phantom 300.

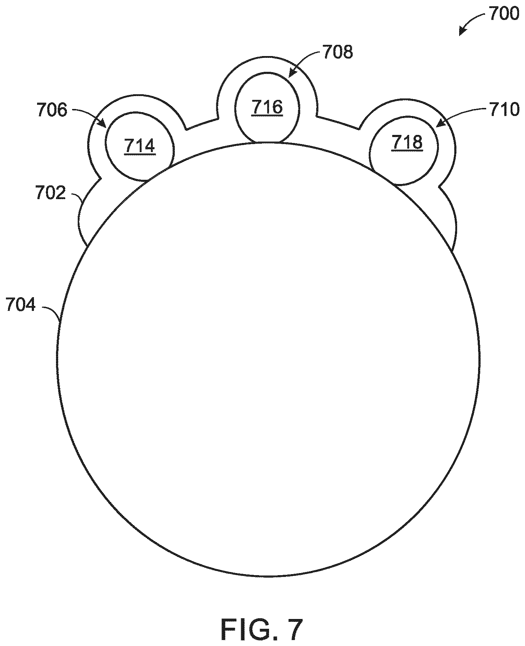

In the illustrated examples of FIGS. 3-6, the first rod holder 312 and the second rod holder 314 are configured to hold one rod, such as the rod 316. In some examples, additionally or alternatively, the first rod holder 312 and the second rod holder 314 may be configured to hold more than one rod. For example, an example rod holder 702 is shown in FIG. 7 coupled to a body 704 of a phantom 700. The rod holder 702 is shown having three openings 706, 708, 710 and coupled to three rods including a first rod 714, a second rod 716, and a third rod 718. Each rod 714, 716, 718 is positioned through one of the openings 706, 708, 710. A second rod holder, identical to the rod holder 702 may also be coupled to the body 704 and the three rods 714, 716, 718. The rod holder 702 may include one or more protrusions, similar to the protrusion 320 of the first rod holder 312. The body 704 may include one or more notches, equal in number to and configured to receive the one or more protrusions of the rod holder 702. In one example, the rod holder 702 may include only one protrusion so that the body 704 may be identical to the body 304. By doing this, the rod holders 312 and 314 depicted in FIGS. 3-5 and the rod holder 702 depicted in FIG. 7 may be interchangeable without modifications to the phantom body 304 and 704.

The rods retained in any embodiments of the rod holders described herein may include an iodinated material. The body of the phantom of any embodiments described herein may include a non-iodinated material. The phantom including each of the iodinated and non-iodinated materials may be used to calibrate an imaging device.

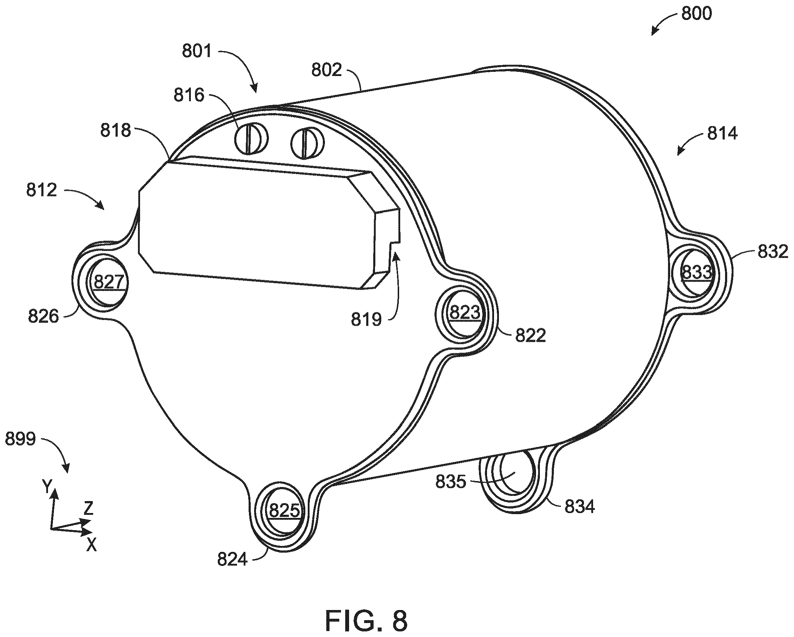

Turning now to FIG. 8, another example phantom 800 including an example rod retention system 801 is depicted.. The phantom 800 may include a body 802. In some examples, the body 802 may include a cylindrical shape. The body 802 may be hollow, forming an interior volume of the cylinder that may contain a material. In one example, the material may be water. In another example, the material in the body 802 may be a different non-iodinated material.

An axis system 899 is shown including three axes, namely an x-axis, a y-axis normal to the x-axis, and a z-axis normal to the x- and y-axes. The z-axis is parallel to a longitudinal length and a central axis of the phantom 800. An x-y plane is parallel to radial directions of the phantom 800.

The retention system 801 may include a first end plate 812 and a second end plate 814 coupled to the body 802 of the phantom 800. The first end plate 812 and the second end plate 814 may be arranged at opposite ends of the body 802. The first end plate 812 may include a substantially circular shape. A plurality of fasteners 816 may physically couple the first end plate 812 to the body 802. In some examples, the first and second end plates 812, 814 may be integrally formed with the body 802. The first end plate 812 may further include a protrusion 818. The protrusion 818 may be arranged relative to the plurality of fasteners 816 such that the plurality of fasteners 816 are between the protrusion 818 and a circumference of the first end plate 812. However, any other locations of the fasteners may be used. The example protrusion 818 may facilitate attachment of the phantom 800 to a table of an imaging device or to a table accessory, allowing the phantom to be scanned for a calibration of the imaging device. As such, the protrusion 818 may include a recess 819, wherein a component of the table may be positioned to attach the phantom 800 to the table or table accessory.

In some examples, the second end plate 84 may be similar to the first end plate 812 in size and shape. That is, the second end plate 814 may include the features of the first end plate 812. As such, the second end plate 814 may include a protrusion 818. The second end plate 814 may further include a plurality of fasteners physically coupling the second end plate 814 to the body 802. Additionally, or alternatively, the second end plate 814 may not include the protrusion such that the phantom 800 may be carried via only the protrusion 818.

The first end plate 812 and the second end plate 814 may further include a plurality of tabs integrally arranged therewith. The first end plate 812 may include a first tab 822, a second tab 824, and a third tab 826. Each of the first tab 822, the second tab 824, and the third tab 826 may be identical and shape and size. The first tab 822, the second tab 824, and the third tab 826 may extend from a circumference of the first end plate 812. In one example, the first tab 822, the second tab 824, and the third tab 826 may be spaced along a lower half of the first end plate 812. In one example, an arc distance between the first tab 822 and the second tab 824 is equal to an arc distance between the second tab 824 and the third tab 826. An arc distance between the first tab 822 and the third tab 826 may be equal to twice the arc distance between the first tab 822 and the second tab 824.

The second end plate 814 may include a first tab 832 and a second tab 834. The second end plate 814 may further include a third tab occluded in the illustration of FIG. 8 due to the body 802. The first tab 832 may be aligned with the first tab 822 along the z-axis. The second tab 834 may be aligned with the second tab 824 along the z-axis. The third tab may be aligned with the third tab 826 along the z-axis. An arc distance between the first tab 832 and the second tab 834 is equal to the arc distance between the first tab 822 and the second tab 824. An arc distance between the second tab 834 and the third tab (e.g., occluded in FIG. 8) may be equal to the arc distance between the second tab 824 and the third tab 826.

Each of the plurality of tabs of the first end plate 812 and the second end plate 814 may include a corresponding opening. That is, the first end plate 812 includes a first set of openings and the second end plate 814 includes a second set of openings. The second set of openings corresponds to the first set of openings. The example openings are configured to receive rods. While each of the first and second end plates 812, 814 of the illustrated example includes three tab and corresponding openings, any number of tabs and corresponding openings may be used. For example, the first tab 822 of the first end plate 812 may include a first opening 823. The second tab 824 of the first end plate 812 may include a second opening 825. The third tab 826 of the first end plate 812 may include a third opening 827. The first tab 832 of the second end plate 814 may include a first opening 833. The second tab 834 of the second end plate 814 may include a second opening 835. The third tab of the second end plate 814 may include a third opening (not shown). The first opening 823 is aligned with the first opening 833. A rod may extend from the first opening 823 to the first opening 833 such that the first tab 822 and the first tab 832 may hold the rod adjacent to the phantom 800. Similarly, the second opening 825 is aligned with the second opening 835 such that an additional rod may extend from the second opening 825 to the second opening 835 such that the second tab 824 and the second tab 834 may hold the further rod with the phantom 800. Similarly, a third rod may extend from the third opening 827 of the first end plate 812 to the third opening of the second end plate 814.

The protrusion 818 may be positioned in an area of the first end plate 812 above a midline of the first end plate 812. In one example, the midline may bisect the first tab 822 and the third tab 826. As such, the protrusion 818 is closer to an outer circumference of the first end plate 812 than the second tab 824.

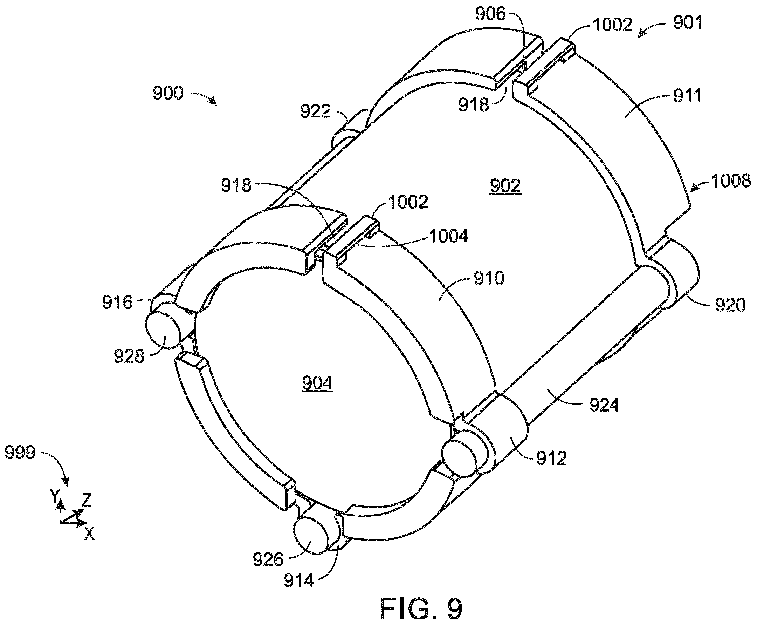

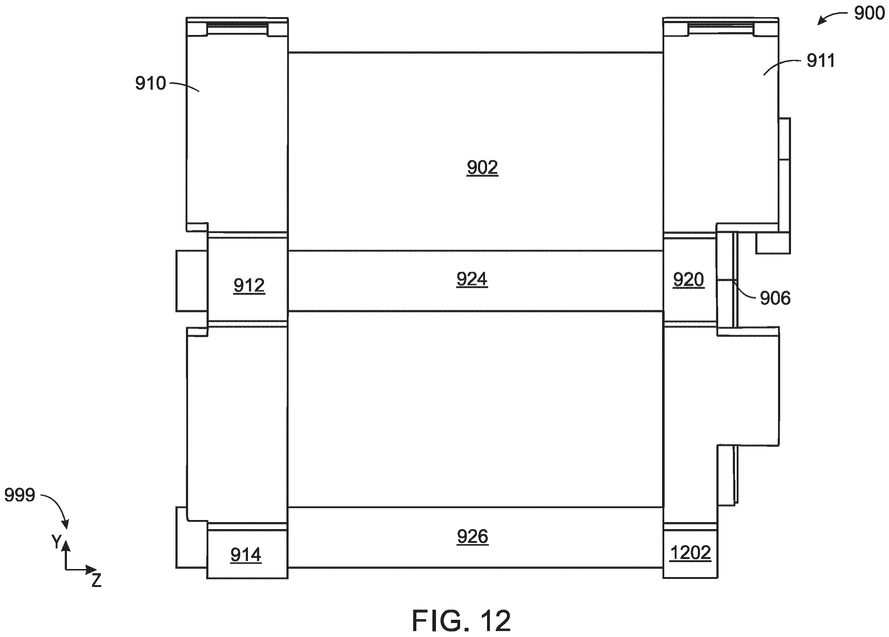

FIG. 9 depicts an alternative example embodiment of a phantom 900 including a rod retention system 901 for use with the example phantom body and rods described herein. An axis system 999 is shown including three axes, namely an x-axis, a y-axis normal to the x-axis, and a z-axis normal to each of the x- and y-axes. The z-axis may be parallel to a longitudinal axis of the phantom 900. An x-y plane may be parallel to a radial directions of the body. Axis system 999 is also shown in FIGS. 12-26.

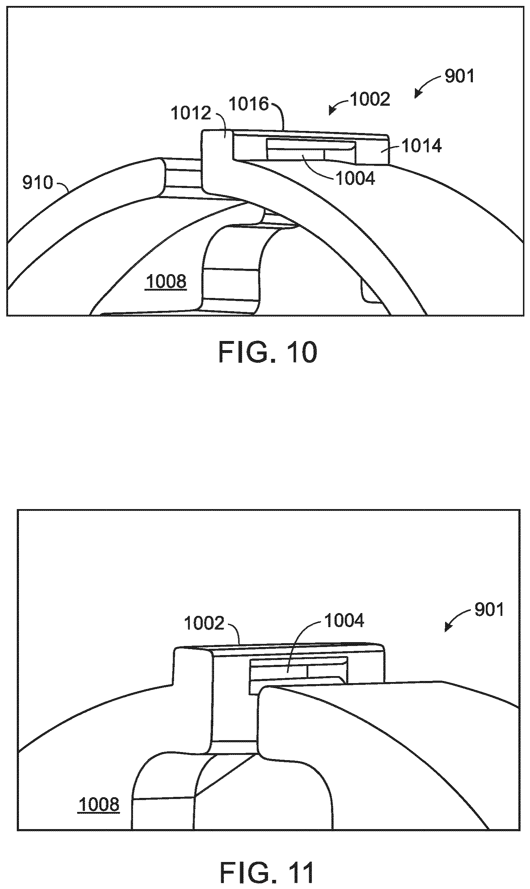

The phantom 900 is shown coupled to a first rod holder 910 and a second rod holder 911 of the rod retention system. The first rod holder 910 and the second rod holder 911 may be similar to one another in shape and size. In the illustrated example, the first and second rod holders 910, 911 are substantially ring-shaped and coupled to opposite ends of an example phantom body 902. The first rod holder 910 and the second rod holder 911 may include one or more of a loop 1002 and an opening 1004, depicted in more detail in FIGS. 10 and 11. Turning now to FIGS. 10 and 11, partial views of a portion of a rod retention system 901 according to an embodiment are depicted. The partial views illustrate a loop 1002 including an opening or slot 1004. The loop 1002 may be coupled to or integrally formed as part of the first rod holder and/or the second rod holder. In one example, the first rod holder and the second rod holder has a circumference based on a circumference of a phantom body 902, as shown in FIGS. 9 and 12-15. A strap or other element may be passed through the opening or slot of the loop 1002 and wrapped around the first or second rod holder. The strap may tighten around the rod holder and fasten the rod holder to the phantom. In this way, the rod holder may be fastened to the phantom via the strap. The loop 1002 may include a first arm 1012, a second arm 1014, and a bridge 1016. The opening 1004 may be shaped via each of the first arm 1012, the second arm 1014, and the bridge 1016. In one example, the first arm 1012 and the second arm 1014 are substantially identical to one another in shape and size. The first arm 1012 and the second arm 1014 may include a rectangular cross-sectional shape. The bridge 1016 may extend from the first arm 1012 to the second arm 1014.

In one example, the strap may include an adhesive. Additionally, or alternatively, the strap may include an interlocking material, a hook and loop element, a webbing, or other fastening system to retain the strap to the phantom. In such examples, a first end of the strap may be secured to rod holder and a second end of the strap may be disposed through the loop and doubled back such that the first end of the strap is coupled to the second end of the strap (e.g., via a hook and loop fastener) to secure the rod holder to the phantom body.

In some examples, rod retention system 901 may include ratchet system, a dial system, or other mechanical system that allows the rod holder to be tightened against an outer surface of the phantom. Some such additional examples may be described further herein. Additionally, or alternatively, the rod retention systems 901 may include a perforations and a prong configured to extend through one of the perforations to strap the rod holder to the phantom.

The first and second rod holder may further include at least one lip 1008 to enable alignment of the first and second rod holders on the phantom body. The lip(s) 1008 are described in greater detail below.

The first rod holder 910 and example may include a plurality of protrusions extending radially from a circumference of the rod holder forming openings in the example first rod holder. For example each rod holder may include a first protrusion 912, a second protrusion 914, and a third protrusion 916. The first protrusion 912, the second protrusion 914, and the third protrusion 916 may be similar to one another in size and shape. The first protrusion 912, the second protrusion 914, and the third protrusion 916 may include a substantially circular shape. Alternatively, any other shape corresponding to a shape of a rod may be suitable. An arc distance between the first protrusion 912 and the second protrusion 914 is equal to an arc distance between the second protrusion 914 and the third protrusion 916. An arc distance between the first protrusion 912 and the third protrusion 916 is approximately twice the arc distance between the first protrusion 912 and the second protrusion 914. An arc distance between the first protrusion 912 and a first end of the rod holder, adjacent to the loop including the slot, is similar to an arc distance between the third protrusion 916 and a second end of the rod holder, opposite the loop. A gap 918 may be positioned between the first end of the rod holder, at which the loop 1002 is arranged, and the second end. A size of the gap 918 may be adjusted based on a tension of the strap or other fastening system coupled to the rod holder. In this way, the rod holder is an incomplete ring such that it is not a complete circle.

The second rod holder 911 may include a plurality of protrusions extending radially from a circumference of the rod holder forming openings in the example second rod holder, similar to those described in conjunction with the first rod holder (shown in FIGS. 12, 13, and 15). The example first rod holder thereby forms a first set of openings and the second rod holder forms a second set of openings. The first and second rod holders are positioned on the body of the phantom such that the first and second set of openings are aligned.

In one example, the first protrusion 912 of the first rod holder 910 is aligned with the first protrusion 920 of the second rod holder 940 along the z-axis. The second protrusion 914 of the first rod holder 910 is aligned with the second protrusion 1202 (shown in FIGS. 12-15) of the second rod holder 911 along the z-axis. The third protrusion 916 of the first rod holder 910 is aligned with the third protrusion 922 of the second rod holder 911.

A first rod 924 may extend from the first protrusion 912 of the first rod holder 910 to the first protrusion 920 of the second rod holder 911. A second rod 926 may extend from the second protrusion 914 of the first rod holder 910 to the second protrusion 1202 of the second rod holder 911. A third rod 928 may extend from the third protrusion 916 of the first rod holder 910 to the third protrusion 922 of the second rod holder 911. The first rod 924, the second rod 926, and the third rod 928 may be substantially identical in size and shape. In one example, each of the first rod 924, the second rod 926, and the third rod 928 are cylindrical and include a similar concentration of materials. In one example, each of the first rod 924, the second rod 926, and the third rod 928 includes iodine. In some examples, additionally or alternatively one or more of the first rod 924, the second rod 926, and the third rod 928 may include a concentration of iodine different than a concentration of iodine included in the other rods. The rods may be replaceable. The rods may be replaced with rods of a different size and/or a different iodine concentration. In some examples, another shape of rods may be used with the example rod holders and rod retention system described herein. In such examples, the openings are shaped to correspond to the shape of the rods.

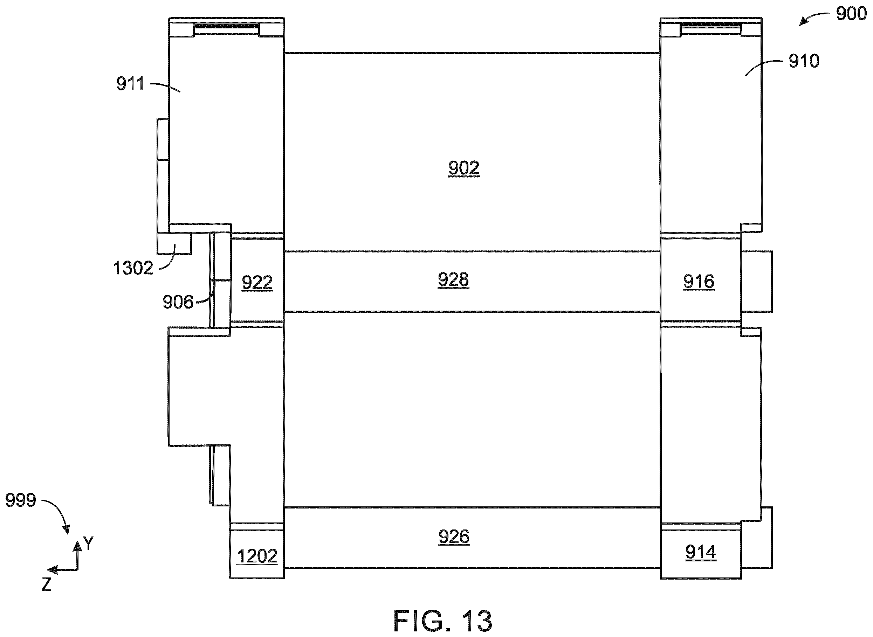

Each of the first rod 924, the second rod 926, and the third rod 928 may be parallel to one another, the z-axis, and to a length of the phantom 900. As shown in FIGS. 12 and 13, the first rod 924, the second rod 926, and the third rod 928 do not extend beyond profiles of the first protrusion 920, the second protrusion 1202, and the third protrusion 922 of the second rod holder 911 along the z-axis. FIGS. 12 and 13 further illustrate where the first rod 924, the second rod 926, and the third rod 928 extend beyond a profile of the first protrusion 912, the second protrusion 914, and the third protrusion 916 of the first rod holder 910. The extension of the first rod 924, the second rod 926, and the third rod 928 may extend beyond an entire profile of the first rod holder 910 include the lips 1008. The lip 1008 may press against end plates of a phantom. The lip(s) 1008 may facilitate alignment such that the rod retention system 901 is positioned in a desired location of the phantom.

The first rod 924, the second rod 926, and the third rod 928 may include a material different than the body 902. For example, the body 902 may include a non-iodinated material and the first rod 924, the second rod 926, and the third rod 928 may include an iodinated material. A concentration of iodine in each of the first rod 924, the second rod 926, and the third rod 928 may be the same or different depending on a desired calibration process. The first rod 924, the second rod 926, and the third rod 928 may be in face-sharing contact with or spaced away from the body 902.

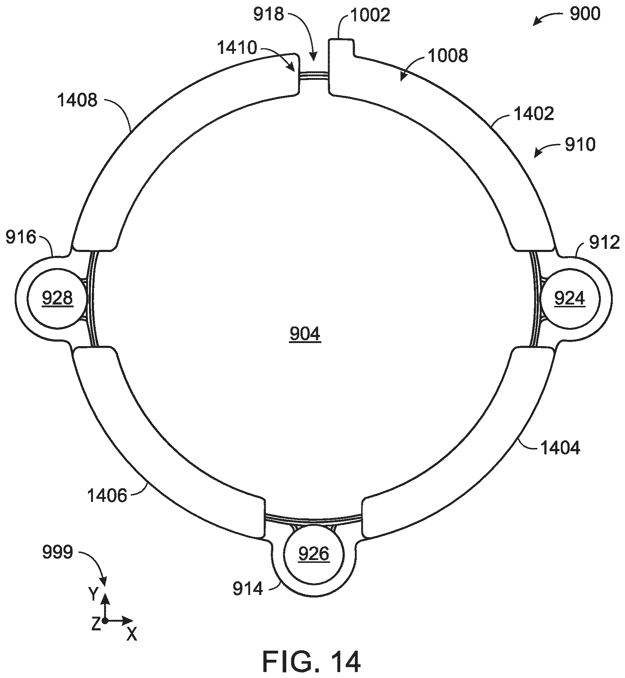

FIG. 14 shows a face-on view of a first end plate 904 of the phantom 900. A lip 1008 of the first rod holder may have a similar depth or thickness along each arc between the respective protrusions 912, 914, 916 along the circumference of the of the first rod holder, as depicted in FIG. 14. The face-on view further illustrates lip of the first rod holder 910, including a first section 1402, a second section 1404, a third section 1406, and a fourth section 1408. In one example, each of the first section 1402, the second section 1404, the third section 1406, and the fourth section 1408 are similar in shape and size. Each of the first section 1402, the second section 1404, the third section 1406, and the fourth section 1408 may include an arc shape. The first section 1402, the second section 1404, the third section 1406, and the fourth section 1408 may be arranged in separate quadrants of the first rod holder 910. The first section 1402 may extend from the first end at which the loop 1002 is arranged to the first protrusion 912. The second section 1404 may extend from the first protrusion 912 to the second protrusion 914. The third section 1406 may extend from the second protrusion 914 to the third protrusion 916. The fourth section 1408 may extend from the third protrusion 916 to the second end 1410 of the first rod holder 910.

The first section 1402, the second section 1404, the third section 1406, and the fourth section 1408 of the lip 1008 may be in face-sharing contact with the first end plate 904. The first section 1402, the second section 1404, the third section 1406, and the fourth section 1408 may block the first rod holder 910 from being positioned along a mid-section of the phantom body 902. Additionally, or alternatively, first section 1402, the second section 1404, the third section 1406, and the fourth section 1408 may facilitate the positioning the first rod holder 910 in a desired position.

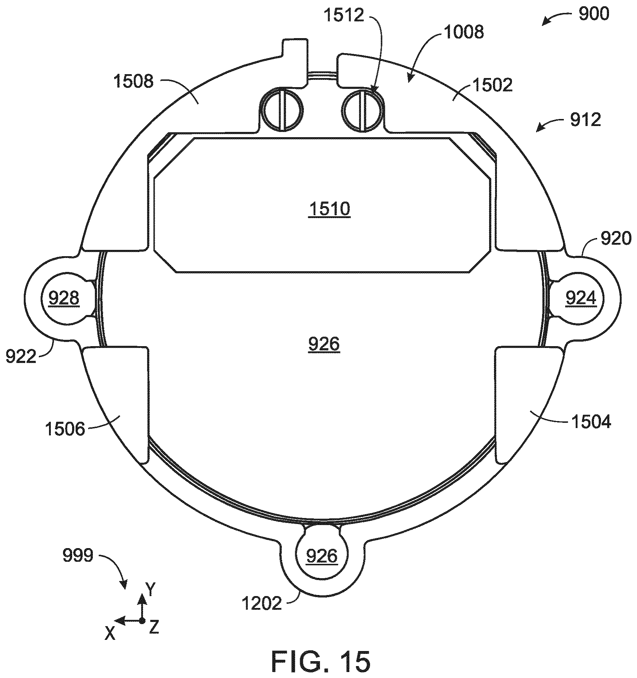

FIG. 15 shows a face-on view of a second end plate 906 of the phantom 900. The second end plate 906 may be arranged at an opposite end of a body 902 of the phantom 900 relative to the first end plate 904. The face-on view further illustrates a lip 1008 of the second rod holder 911, including a first section 1502, a second section 1504, a third section 1506, and a fourth section 1508. The first section 1502, the second section 1504, the third section 1506, and the fourth section 1508 of the lip may be arranged in separate quadrants of the second rod holder 911. For example, a lip 908 of the second rod holder, as depicted in FIG. 15 may include additional cutouts to accommodate a protrusion 1510 of the phantom.

The first section 1502, the second section 1504, the third section 1506, and the fourth section 1508 may be in face-sharing contact with the second end plate 906. The first section 1502, the second section 1504, the third section 1506, and the fourth section 1508 may prevent the second rod holder 911 from being positioned along a mid-section of the phantom body 902. Additionally, or alternatively, first section 1502, the second section 1504, the third section 1506, and the fourth section 1508 may help facilitate positioning the second rod holder 911 in a desired position.

The first section 1502 and the fourth section 1508 may be shaped similarly to one another. The second section 1504 and the third section 1506 may be shaped similarly to one another and different than the first section 1502 and the fourth section 1508. The first section 1502 and the fourth section 1508 may be shaped complementarily to a protrusion 1510 and a plurality of fasteners 1512. In one example, the first section 1502 and the fourth section 1508 may include a first indentation. The first indentation may be curved to match a curvature of the plurality of fasteners 1512. The first section 1502 and the fourth section 1508 may further include a second indentation. The second indentations may include linear sides that match a shape of the protrusions 1510. As such, the second indentations may follow a shape of the protrusions 1510 without contacting the protrusions 1510.

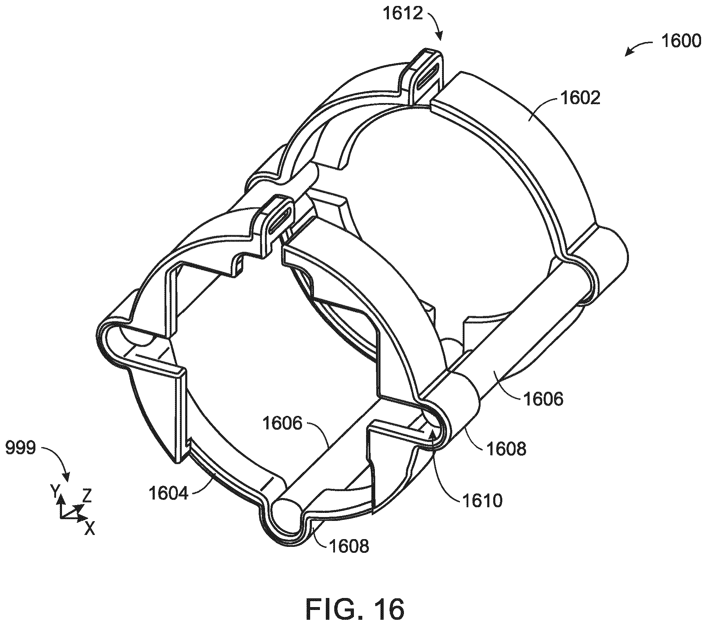

FIG. 16 shows a perspective view of a rod retention system 1600 for a phantom. The rod retention system 1600 may be coupled to a body, which may be similar to the body 1102 introduced above with reference to FIGS. 11-15. In the example of FIG. 16, the rod retention system 1600 includes a first rod holder 1602, a second rod holder 1604, and a plurality of rods 1606. The first rod holder 1602 and the second rod holder 1604 include a plurality of protrusions 1608 including openings 1610. In the example shown in FIG. 16, each rod of the plurality of rods may be press-fit into an opening of a protrusion Similar to the phantom 900, in the example shown in FIG. 16, the first rod holder 1602 and second rod holder 1604 are configured to be strapped to the body of a phantom via a loop and strap mechanism 1612. However, in other examples, other tightening mechanisms may be suitable.

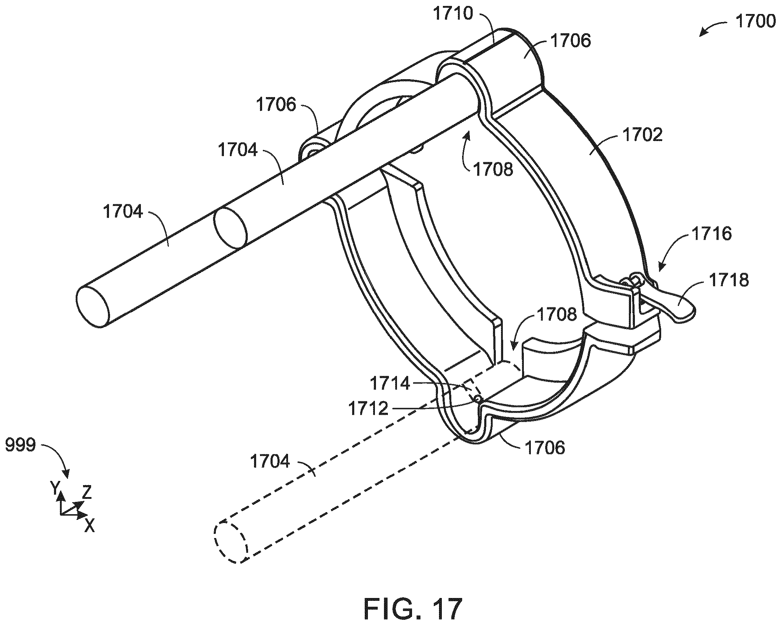

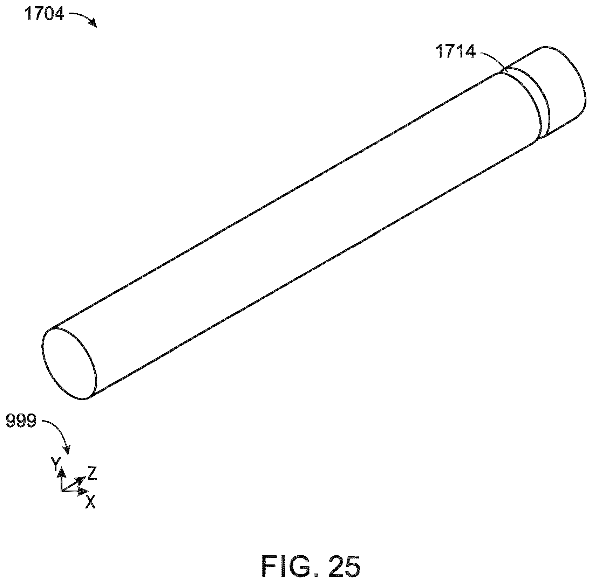

FIG. 17 shows a perspective view of a rod retention system 1700 for a phantom. The rod retention system 1700 may be coupled to a body, such as a body similar to the body 902 introduced above with reference to FIGS. 11-15. In the example of FIG. 17, the rod retention system 1700 includes a first rod holder 1702 and a plurality of rods 1704. The first rod holder 1702, and the other examples of rod holders that follow in FIGS. 17-24, may be examples of a rod retention system configured to hold at least one rod 1704 to the outer surface of the body of the phantom. The first rod holder 1704 includes a plurality of protrusions 1706 including openings 1708. In some examples, at least one protrusion 1706 may include an alignment feature 1710 (e.g., a laser alignment line), which may be used with a laser alignment tool of the imaging device to align the phantom within the bore or on the table. Further, each protrusion 1706 may include a detent 1712 corresponding with a groove 1714 on the at least one rod. In the figure, one of the rods 1704, including the groove 1714 are depicted in dashed lines so that the detent 1712 is visible. The detent 1712 and groove 1714 secure the at least one rod 1704 in place within the rod holder 1702. In some examples, the detent 1712 may be a spring-loaded button which depresses in response to pressure, to interface with a radial groove 1714 positioned adjacent an end of each rod 1704. An example of a spring-loaded button is shown schematically as a circle positioned on the inside surface of an opening 1708. Another example of a rod 1704 with a radial groove 1714 is shown in FIG. 25. In one example, each rod 1704 of the plurality of rods 1704 may be slid longitudinally into the opening 1708 of a protrusion 1706 of the plurality of protrusions 1706 of the rod holder 1702. As the rod slides into the openings 1708 of the protrusions 1706, the rod 1704 depresses the spring-loaded button 17112 until the button and radial groove 1714 align, whereupon the pressure releases and the button expands into the radial groove. In this way, the rod 1704 may be held in the clamp. In some examples, an end of the rod 1704 may include a tapered portion to facilitate depression of the detent 1712 as the rod 1704 is inserted into the opening 1708 of the protrusion 1706. Further, the first rod holder 1702 includes a fastener 1716 or clasp that is configured to adjustably hold the first rod holder to the body. In the illustrated example, the fastener or clasp 1716 may be a screw with a locking lever 1718. That is, the lever 1718 can be turned by hand (e.g., by a technician) to tighten the rod holder 1702around the circumference of the body. The lever 1718 may then be moved to a lock position (e.g., perpendicular to the screw) to prevent further movement of the screw to hold the secure the fastener and, as a result, the rod holder in place. In other examples, a screw, nut, clamp, a wingnut or other tightening mechanisms may be suitable.

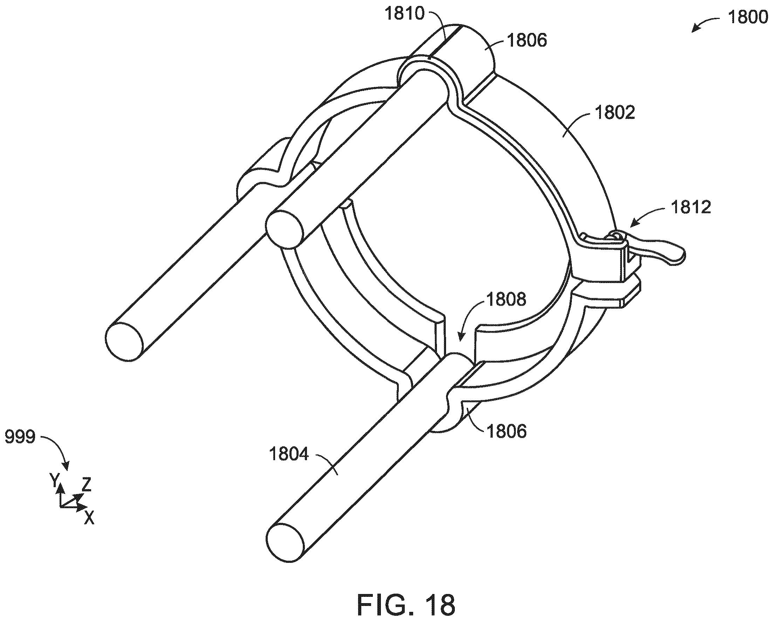

FIG. 18 shows a perspective view of a rod retention system 1800 for a phantom. The rod retention system 1800 may be coupled to a body, which may be similar to the body 902 introduced above with reference to FIGS. 11-15. In the example of FIG. 18, the rod retention system 1800 includes a first rod holder 1802 and a plurality of rods 1804. The first rod holder 1802 includes a plurality of protrusions 1806, which may be configured similarly to the plurality of protrusions introduced above with reference to FIG. 17, where each rod of the plurality of rods 1804 may be pressed into an opening 1808 of a protrusion 1806 via squeeze compression. In some examples, at least one protrusion 1801 may include a laser alignment line 1810, which may be used with a laser alignment tool to align the phantom. Similar to the rod retention system 1700, in the example shown in FIG. 17, the first rod holder 1802 is configured to be adjustably held to the body via a clasp or fastener 1812. However, in other examples, other tightening mechanisms may be suitable.

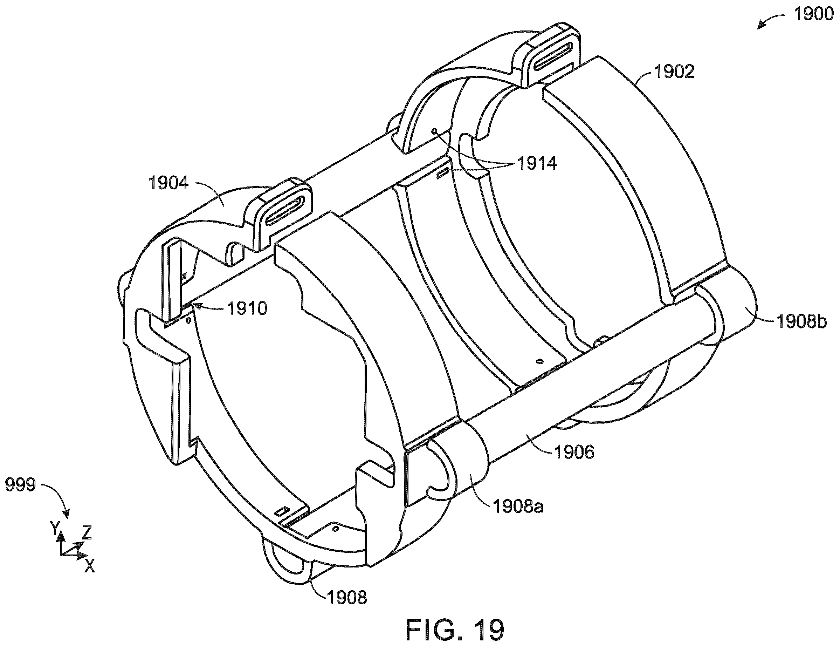

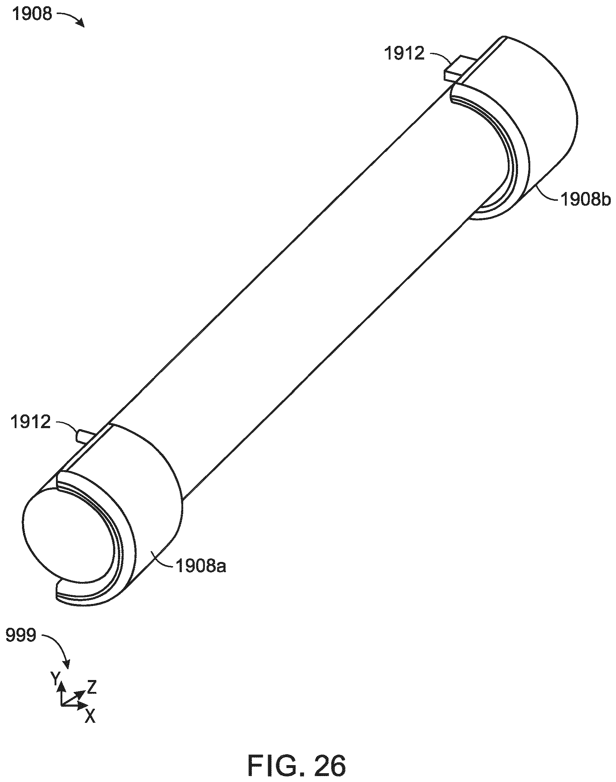

FIG. 19 shows a perspective view of a rod retention system 1900 for a phantom. The rod retention system 1900 may be coupled to a body, which may be similar to the body 902 introduced above with reference to FIGS. 11-15. In the example of FIG. 19, the rod retention system 1900 includes a first rod holder 1902, a second rod holder 1904, and a plurality of rods 1906. Each rod 1906 of the plurality of rods 1906 comprises at least one rod clip 1908, where the rod clip 1908 is integral with rod 1906. Alternatively, the rod clip 1908 may be removably coupled to the rods 1906 via, for example, a press-fit connection, adhesive, magnets, etc. The rod clips 1908 are configured to removably attach to the rod holder 1902, 1904, e.g., to clip on and clip off. The example rod clips 1908 are configured to fit over openings 1910 in the first and second rod holders 1902, 1904. For example, the rod clips 1908 may include projections 1912 (depicted in FIG. 26) that interlock with corresponding slots or apertures 1914 arranged on the rod holder 1902, 1904. In some examples, the projections 1912 are shaped (e.g., have different shapes) such that the rod 1906 can only be attached in one orientation. In the example of FIG. 19, each rod 1906 includes a first rod clip 1908a and a second rod clip 1908b, the first and second rod clips 1908a, 1908b arranged at ends of the rod 1906. An example of a rod 1906 configured with a first rod clip 1908a and a second rod clip 1908b is shown in FIG. 26. In other examples, the rod clips may magnetically attach to the rod holder. Similar to the phantom 900, in the example shown in FIG. 9, the first rod holder 1902 and second rod holder 1904 are configured to be strapped to the body via a loop and strap mechanism. However, in other examples, other tightening mechanisms may be suitable.

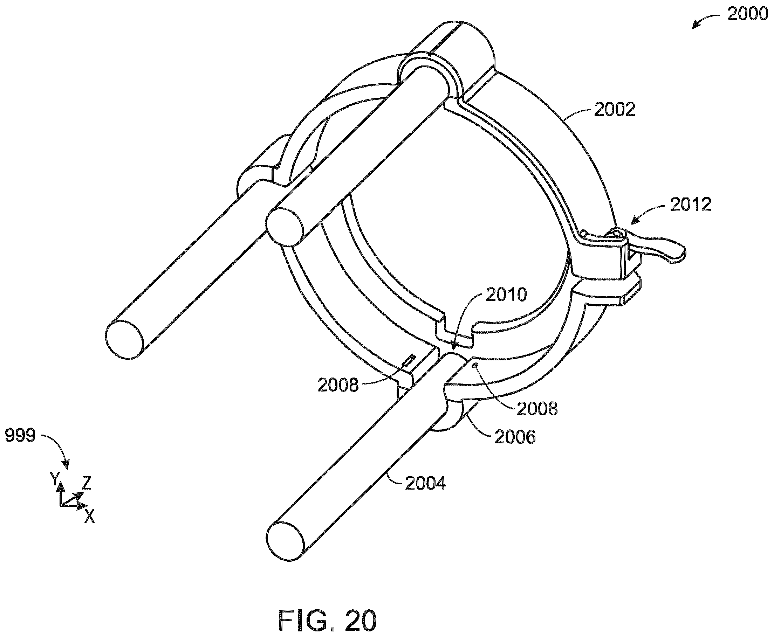

FIG. 20 shows a perspective view of a rod retention system 2000 for a phantom. The rod retention system 2000 may be coupled to a body, which may be similar to the body 902 introduced above with reference to FIGS. 11-15. In the example of FIG. 20, the rod retention system 2000 includes a first rod holder 2002 and a plurality of rods 2004. Similar to the example of FIG. 19, each rod 2004 of the plurality of rods 2004 comprises a rod clip 2006, where the rod clip 2006 is integral with rod 2004 or otherwise coupled to the rod 2004. The rod clips 2006 are configured to removably attach to the rod holder 2002, e.g., to clip on and clip off via projections 1912 (depicted in FIG. 26) on the rod 2004, similar to those described in conjunction with FIG. 19, and apertures 2008 adjacent openings 2010 of the rod holder 2002. Similar to the rod retention system 1700, in the example shown in FIG. 17, the first rod holder 2002 is configured to be adjustably held to the body via a clasp or fastener 2012. However, in other examples, other tightening mechanisms may be suitable.

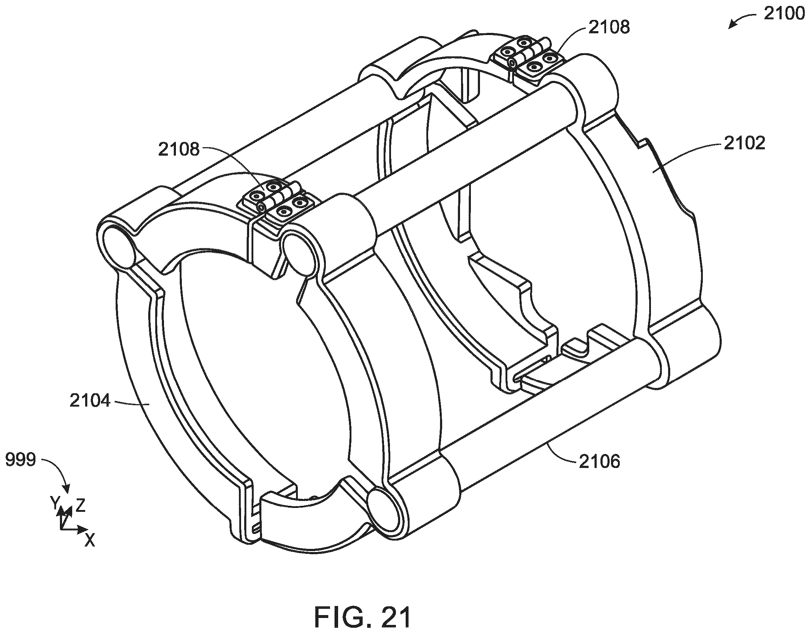

FIG. 21 shows a perspective view of a rod retention system 2100 for a phantom. The rod retention system 2100 may be coupled to a body, which may be similar to the body 902 introduced above with reference to FIGS. 11-15. In the example of FIG. 21, the rod retention system 2100 includes a first rod holder 2102, a second rod holder 2104, and a plurality of rods 2106. The first rod holder 2102 and the second rod holder 2104 each include a hinge 2108, where the hinge 2108 is configured to rotate about a hinge axis that is parallel to a longitudinal or central axis of the rod retention system 2100. In the example of FIG. 21, the hinge 2108 is an external hinge positioned on the outside of the rod holder 2102, 2104. As an example, the first rod holder 2102 and second rod holder 2104 may be opened by rotation around the hinge 2108, e.g., like a clam shell, and the plurality of rods 2106 placed into a plurality of protrusions, e.g., similar to the clamps described with reference to FIGS. 11-15, may be press-fit, or otherwise attached with rod clips, such as the rod clips described in conjunction with FIG. 19. With the rods 2106 in place, the rod holders 2102. 2104 may be closed around the body of a phantom, such as body 902. Similar to the phantom 900, in the example shown in FIG. 21, the first rod holder 2102 and second rod holder 2104 are configured to be strapped to the body via a loop and strap mechanism. However, in other examples, other tightening mechanisms may be suitable.

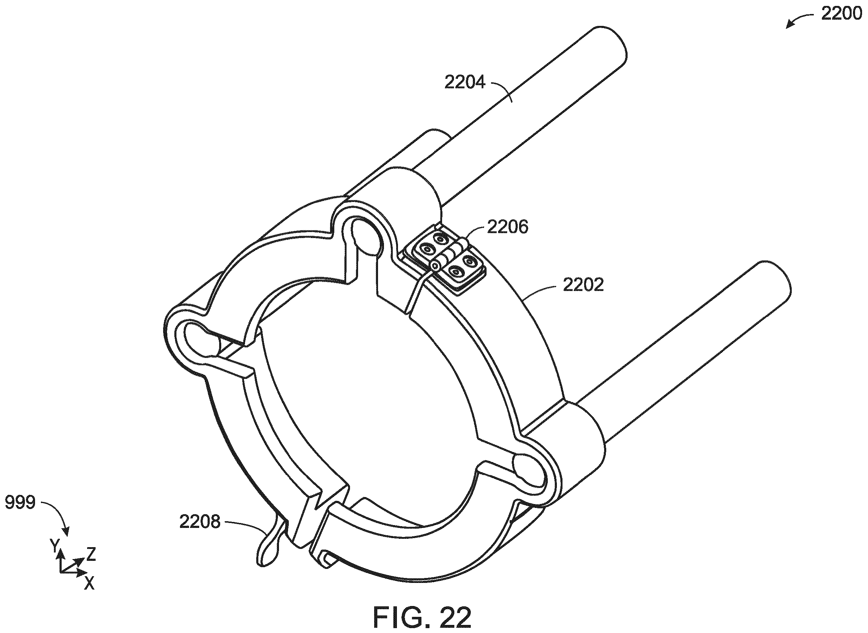

FIG. 22 shows a perspective view of a rod retention system 2200 for a phantom. The rod retention system 2200 may be coupled to a body, which may be similar to the body 902 introduced above with reference to FIGS. 11-15. In the example of FIG. 22, the rod retention system 2200 includes a first rod holder 2202 and a plurality of rods 2204. The first rod holder 2202 includes a hinge 2206 that is similar to the hinge 2108 described above with reference to FIG. 21, e.g., an external hinge. Similar to the rod retention system 1600, in the example shown in FIG. 22, the first rod holder 2202 is configured to be adjustably held to the body via a clasp or fastener 2208. However, in other examples, other tightening mechanisms may be suitable.

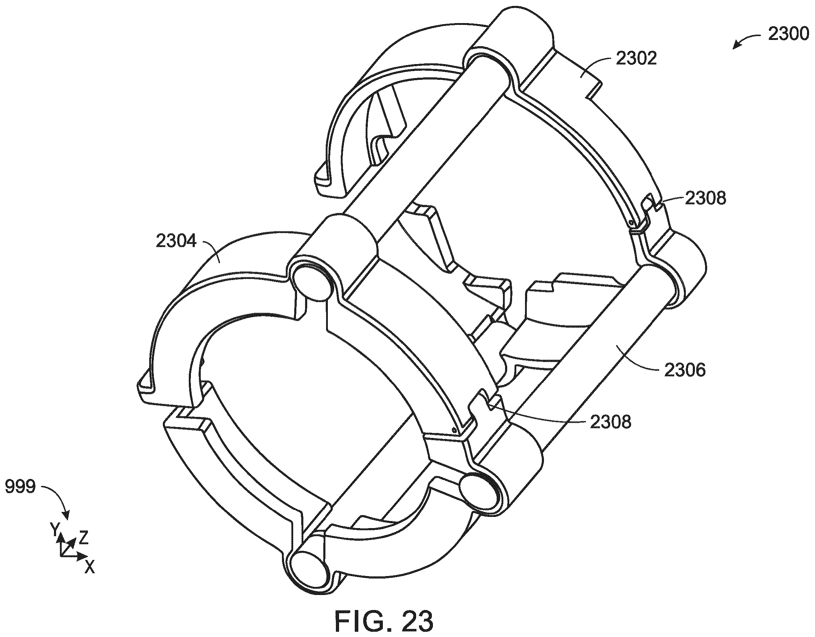

FIG. 23 shows a perspective view of a rod retention system 2300 for a phantom. The rod retention system 2300 may be coupled to a body, which may be similar to the body 902 introduced above with reference to FIGS. 11-15. In the example of FIG. 23, the rod retention system 2300 includes a first rod holder 2302, a second rod holder 2304, and a plurality of rods 2306. The first rod holder 2302 and the second rod holder 2304 each include a hinge 2308, which functions in a similar manner to the hinge 2108, 2206 described above with reference to FIGS. 21-22. However, in the example of FIG. 23, the hinge 2308 is an internal hinge. Similar to the phantom 900, in the example shown in FIG. 23, the first rod holder 2302 and second rod holder 2304 are configured to be strapped to the body via a loop and strap mechanism. However, in other examples, other tightening mechanisms may be suitable.

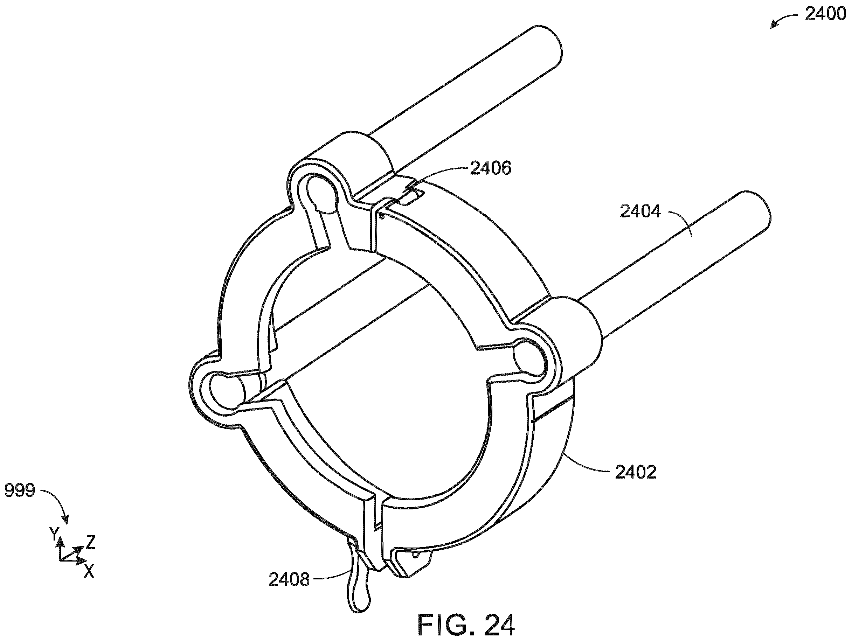

FIG. 24 shows a perspective view of a rod retention system 2400 for a phantom. The rod retention system 2400 may be coupled to a body, which may be similar to the body 902 introduced above with reference to FIGS. 11-15. In the example of FIG. 24, the rod retention system 2400 includes a first rod holder 2402 and a plurality of rods 2404. The first rod holder 2402 includes a hinge 2406 that is similar to the hinge 2308 described above with reference to FIG. 23, e.g., an internal hinge. Similar to the rod retention system 1600, in the example shown in FIG. 24, the first rod holder 2402 is configured to be adjustably held to the body via a clasp or fastener 2408. However, in other examples, other tightening mechanisms may be suitable. While FIGS 22-24 depict various types of hinges, it may be understood that the location of the hinge may vary. For example, in FIG. 22, the hinge is positioned opposite the clasp or fastener (e.g., 180 degrees from the fastener). Alternatively, the hinge is positioned at a different point around the circumference of the rod holder. For example, FIG. 23 depicts an example hinge located adjacent to a second protrusion of the rod holder, which positioned opposite the fastener or clasp. Other positions of the hinge may be suitable.

FIG. 25 and FIG. 26 each show an example of a rod, such an iodinated rod. Turning first to FIG. 25, a rod 1704 is shown. The rod 1704 is an example of a rod 1704 comprising a radial groove 1714. The rod 1704 with radial groove 1714 may be configured to slide into a protrusion of a rod holder comprising a corresponding spring-loaded button, such as described above with reference to FIG. 17. FIG. 26 shows a rod 1906. The rod 1906 is an example of a rod comprising at least one rod clip 1908, where the rod clip 1908 is integral with rod 1906. In the example, the rod 1906 includes a first rod clip 1908a arranged at a first end and a second rod clip 1908b arranged at an opposing, second end. The rod clips 1908 are configured to removably attach to a rod holder, e.g., to clip on and clip off, such as described above with reference to FIGS. 19-20. As shown in the example, the rod clips 1908 may include projections 1912 or protrusions that interlock with corresponding slots or apertures on the rod holder.

In this way, at least a first rod holder is included in a rod retention system configured to hold iodinated rods. The first rod holder, and in some examples a second rod holder or a plurality of rod holders, may interlock with features of a body of a phantom. Additionally, or alternatively, the first rod holder and the second rod holder may be integrally arranged with a first end plate and a second end plate of the phantom. Additionally, or alternatively, the at least a first rod holder, and in some examples the second rod holder, may be strapped or physically coupled to outer surface of the phantom without interlocking with a feature of the phantom. As such, the first rod holder and, when included, the second rod holder, may be retrofitted to pre-existing phantoms without modifications to the phantom design.

The at least a first rod holder may include a flexible and/or a rigid material. In one example, the rod holder, or plurality of rod holder, may receive one or more of the plurality of iodinated rods prior to being coupled to the phantom. Additionally, or alternatively, the at least a first rod holder may be coupled to the phantom prior to receiving one or more of the plurality of iodinated rods. Each of the plurality of iodinated rods may be slid through corresponding eyelets of the at least a first rod holder.

The at least a first rod holder may include eyelets and/or openings shaped to receive the iodinated rods. The eyelets may extend away from the body. As such, the rods may be positioned radially outside of an outer surface of the body. In some examples, a gap may be arranged between the outer surface of the body and the plurality of iodinated rods such that the iodinated rods do not physically touch the body. In other embodiments, additionally or alternatively, the gap may be omitted and the plurality of rods may physically touch the body.

In some examples, the body may be a primary cylinder and the plurality of rods may be smaller cylinders positioned about the body. A spacing of the plurality of rods may be fixed. In some embodiments, the plurality of rods may be held within a same quadrant of the phantom. In other embodiments, the plurality of rods may be held within a same half of the phantom. In some applications, one or more eyelets of the plurality of rod holders may not be used such that rods are only held by some and not all of the plurality of eyelets. If the plurality of iodinated rods is undesired, the plurality of rod holders may be decoupled from the phantom. Decoupling may include sliding, unfastening, or unstrapping the at least a first rod holder in some examples.

The disclosure also provides support for a retention system for a phantom for calibrating an imaging system, the retention system comprising: at least one rod holder, wherein the at least one rod holder includes: at least one opening, wherein the at least one opening is configured to secure at least one rod of the phantom in the retention system, and a fastener, wherein the fastener is configured to secure the at least one rod holder to a body of the phantom. In a first example of the system, the at least one rod holder includes a plate coupled to an end of the body of the phantom, wherein the at least one opening is integrally formed with the plate. In a second example of the system, optionally including the first example, the at least one rod holder includes a ring, wherein the ring is to be coupled around an outer surface of the body of the phantom. In a third example of the system, optionally including one or both of the first and second examples, the fastener of the at least one rod holder includes a detent that interlocks with a groove of the body of the phantom. In a fourth example of the system, optionally including one or more or each of the first through third examples, the fastener of the at least one rod holder includes a screw. In a fifth example of the system, optionally including one or more or each of the first through fourth examples, the screw includes a locking lever to facilitate tightening and locking the screw. In a sixth example of the system, optionally including one or more or each of the first through fifth examples, the fastener includes a slot configured to receive a strap to secure the at least one rod holder around the outer surface of the body. In a seventh example of the system, optionally including one or more or each of the first through sixth examples, the at least one rod holder includes at least one aperture to receive a at least one detent of a clip coupled to a rod, wherein the clip enables the rod to be removably coupled to the at least one rod holder. In a eighth example of the system, optionally including one or more or each of the first through seventh examples, the at least one rod holder includes a hinge. In a ninth example of the system, optionally including one or more or each of the first through eighth examples, the at least one rod holder includes at least one detent corresponding with a groove on the at least one rod, wherein the at least one detent and at least one groove secure the at least one rod in place within the rod holder.

The disclosure also provides support for a phantom for an imaging system, comprising: a body, one or more rods, and a rod retention system coupled to an outer surface of the body of the phantom, wherein the rod retention system includes a plurality of openings, wherein each of the plurality of openings is to receive one of the rods, wherein each of the plurality of openings includes a central axis parallel with a longitudinal axis of the body. In a first example of the system, the one or more rods includes an iodinated material, and wherein the body comprises a non-iodinated fluid. In a second example of the system, optionally including the first example, the rod retention system includes a fastener to couple the rod retention system the body. In a third example of the system, optionally including one or both of the first and second examples, the plurality of openings of the rod retention system includes a first set of openings and a second set of openings positioned adjacent opposite ends of the body, wherein each opening of the first set aligns with an opening of the second set. In a fourth example of the system, optionally including one or more or each of the first through third examples, each of the one or more rods incudes a first end positioned through one of the first set of openings and a second end positioned through a corresponding one of the second set of openings.

The disclosure also provides support for a rod retention system for a phantom for calibrating an imaging system, the rod retention system comprising: at least one rod holder configured to be removably coupled to a phantom body, and at least one rod configured to be removably coupled to the at least one rod holder, wherein the at least one rod includes an integrated locking mechanism. In a first example of the system, the at least one rod holder includes at least one aperture, and wherein the integrated locking mechanism includes at least one detent to interface with the at least one aperture to couple the at least one rod to the rod holder. In a second example of the system, optionally including the first example, the at least one detent is positioned on a clip of the at least one rod. In a third example of the system, optionally including one or both of the first and second examples, the at least one rod holder includes at least one detent corresponding with a groove on the at least one rod, wherein the at least one detent and at least one groove secure the at least one rod in place within the rod holder. In a fourth example of the system, optionally including one or more or each of the first through third examples, the at least one rod holder includes at least one alignment feature.

FIGS. 1-26 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example. It will be appreciated that one or more components referred to as being "substantially similar and/or identical" differ from one another according to manufacturing tolerances (e.g., within 1-5% deviation). FIGS. 3-26 are shown approximately to scale, however, other dimensions may be used if desired.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A retention system for a phantom for calibrating an imaging system, the retention system comprising:
at least one rod holder, wherein the at least one rod holder includes:
at least one opening, wherein the at least one opening is configured to secure at least one rod of the phantom in the retention system; and
a fastener, wherein the fastener is configured to secure the at least one rod holder to a body of the phantom.

2. The retention system of claim 1, wherein the at least one rod holder includes a plate coupled to an end of the body of the phantom, wherein the at least one opening is integrally formed with the plate.

3. The retention system of claim 1, wherein the at least one rod holder includes a ring, wherein the ring is to be coupled around an outer surface of the body of the phantom.

4. The retention system of claim 1, wherein the fastener of the at least one rod holder includes a detent that interlocks with a groove of the body of the phantom.

5. The retention system of claim 1, wherein the fastener of the at least one rod holder includes a screw.

6. The retention system of claim 5, wherein the screw includes a locking lever to facilitate tightening and locking the screw.

7. The retention system of claim 1, wherein the fastener includes a slot configured to receive a strap to secure the at least one rod holder around an outer surface of the body.

8. The retention system of claim 1, wherein the at least one rod holder includes at least one aperture to receive a at least one detent of a clip coupled to a rod, wherein the clip enables the rod to be removably coupled to the at least one rod holder.

9. The retention system of claim 1, wherein the at least one rod holder includes a hinge.

10. The retention system of claim 1, wherein the at least one rod holder includes at least one detent corresponding with a groove on the at least one rod, wherein the at least one detent and at least one groove secure the at least one rod in place within the rod holder.

11. A phantom for an imaging system, comprising:
a body;
one or more rods; and
a rod retention system coupled to an outer surface of the body of the phantom, wherein the rod retention system includes a plurality of openings, wherein each of the plurality of openings is to receive one of the rods, wherein each of the plurality of openings includes a central axis parallel with a longitudinal axis of the body.

12. The phantom of claim 11, wherein the one or more rods includes an iodinated material, and wherein the body comprises a non-iodinated fluid.

13. The phantom of claim 11, wherein the rod retention system includes a fastener to couple the rod retention system the body.

14. The phantom of claim 11, wherein the plurality of openings of the rod retention system includes a first set of openings and a second set of openings positioned adjacent opposite ends of the body, wherein each opening of the first set aligns with an opening of the second set.

15. The phantom of claim 14, wherein each of the one or more rods incudes a first end positioned through one of the first set of openings and a second end positioned through a corresponding one of the second set of openings.
